# EUROPEAN PATENT SPECIFICATION

(11) **EP 0 448 213 B1**
(45) Date of publication and mention of the grant of the patent: **15.05.1996**
(21) Application number: 91301108.6
(22) Date of filing: 12.02.1991
(51) Int. Cl.: A61K 31/20, A61K 31/07

(54) **The use of retinoids to minimize skin damage**
Verwendung von Retinoiden zur Verringerung von Hautbeschädigung
L'utilisation des rétinoides pour amoindrir les endommagements de la peau

(30) Priority: 12.02.1990 US 478817
(43) Date of publication of application: 25.09.1991
(73) Proprietor: E.R. SQUIBB & SONS, INC., Princeton New Jersey 08543-4000 (US)
(72) Inventor: Shannon, Ronald J., Doylestown, PA (US); Bolton, Laura L., Metuchen, NJ (US)
(74) Representative: Thomas, Roger Tamlyn

(56) References cited:
- EP-A- 0 253 393
- EP-A- 0 266 992
- EP-A- 0 339 905
- US-A- 3 896 789
- US-A- 3 966 967
- US-A- 4 073 291

## Description

Retinoids have been recognized to be essential for the normal growth and differentiation of epithelial cells. Hung, et al. "Topical Trentinoin and Epithelial Wound Healing", Arch Dermatol, 125, p. 65 - 69 (1989) reported that pretreatment with 0.05% tretinoin cream daily acelerated reepithelialization of dermabrasion wounds as indicated by the complete absence of crust and eschar.

Bryce et al., "Retinoic Acids Promote the Repair of the Dermal Damage and the Effacement of Wrinkles in the UVB-Irradiated Hairless Mouse", J. Invest. Dermatology, 91, p. 175 - 180 (1988) report that topically applied retinoic acids led to the complete effacement of permanent wrinkles and that the process appears to be permanent.

L.H. Kligman, "Preventing, Delaying and Repairing Photoaged Skin", Cutis, 41, p. 419 - 420 (1988), reports that retinoids enhance the repair of photodamaged skin that is protected from further sun exposure. A. M. Kligman in European Patent Application 253,393 discloses that various effects of photoaging of sundamaged skin including impairment of differentiation of epidermal epithelial cells and loss of collagen fibers, abnormal changes in elastic fibers and the deterioration of small blood vessels in the dermis of the skin are retarded by topical application of retinoids.

Kligman et al. in European Patent Application 266,992 and U.S. Patent 4,889,847 disclose a method of preventing glucocorticoid-induced skin atrophy by the topical administration of retinoids.

Kligman in U.S. Patent 3,729,568 discloses treating acne by the topical administration of vitamin A acid.

Mezick in U.S. Patent 4,487,782 discloses treating non-inflammatory acne by the topical administration of 13-cis-retinoic acid.

Kwan-Hua Lee in U.S. Patents 3,689,667, 3,882,244, 3,966,967 and 4,048,204 disclose various retinoic acid analogs that are effective in promoting wound healing.

Gander et al. in U.S. Patents 4,055,659, 4,126,693, 4,126,697, 4,126,698, 4,126,699, 4,129,662, and 4,304,787 disclose esters and amides of all-trans-retinoic acid which are useful in the treatment of acne.

Papa in U.S. Patent 4,214,000 discloses a zinc salt of all-trans-retinoic acid which is described as having anti-acne activity but with less tendency to cause flaking or irritation than retinoic acid.

Van Scott et al. in U.S. Patent 3,932,665 discloses treating acne by the topical administration of retinal.

Parish et al. in U.S. Patent 4,677,120 disclose various esters and amides of 13-cis-retinoic acid useful in the treatment of acne and skin diseases.

DeLuca et al. in U.S. Patent 4,757,140 disclose N-hydroxysuccinimidyl esters of all-trans and 13-cis-retinoic acid. The compounds are disclosed as having utility in treating acne and ichthyosis.

Felty et al. in U.S. Patent 3,906,108 discloses a stabilized tretinoin cream emulsion. Scott et al. in U.S. Patent 4,727,088 disclose a pharmaceutical preparation in a semi-solid vehicle containing a retinoid, a volatile silicone, a fatty alcohol containing 12 to 22 carbons, preservatives and emulsifying agents. Tranclk in U.S. Patent 3,896,789 disclose applying retinoic acid by incorporating the retinoic acid and a stabilizer within the pressure-sensitive adhesive layer of an adhesive tape.

According to one aspect of the invention, there is provided the use of a retinoid for the manufacture of a medicament for preventing or lessening the skin damaging effects of pressure sores and ischemic ulcers by pretreatment of areas of the body susceptible to the formation of pressure sores and ischemic ulcers.

According to another aspect of the invention, there is provided the use of a retinoid for the manufacture of a medicament for preventing or lessening skin damage due to the stripping of the epidermal layer caused by the removal of adhesives from the body by pretreatment of the areas of the body from which an adhesive will be removed.

This invention is directed to the use of retinoids to condition areas of a patient's body that are susceptible to the formation of pressure sores and ischemic ulcers. The pretreatment with retinoids lessens the likelihood that such sores and ulcers will develop. It also limits the severity of skin damage should an ulcer develop. In addition, this invention is also directed to the use of retinoids to condition areas of a patient's body and lessen or prevent damage due to skin stripping that results when adhesive backed tapes, dressing, or medical devices are removed.

The retinoid compound is administered topically, for example, in a cream, lotion, solution, ointment, or hydrogel formulation or by coating the retinoid compound on the skin contacting adhesive surface of a bandage or dressing, by incorporating the retinoid compound within the adhesive layer of a bandage or dressing, or by incorporating the retinoid at the skin contacting adhesive surface and adjacent strata of the bandage or dressing.

Further preferred features of the invention will now be described.

The term retinoid includes all natural and synthetic analogs of vitamin A or retinol-like compounds which possess the biological activity of vitamin A in the skin. Suitable retinoids include:
retinol (vitamin A);
retinal (vitamin A aldehyde);
all-trans-retinoic acid (vitamin A acid, tretinoin);
13-cis-retinoic acid;
(all-E)-9-(4-methoxy-2,3,6-trimethylphenyl)-3,7-dimethyl-2,4,6,8-nonatetraenoic acid and its ethyl ester;
N-ethyl-9-(4-methoxy-2,3,6-trimethylphenyl)-3, 7-dimethyl-2,4,6,8-nonatetraenamide;
(E,E)-9-(2,6-dichloro-4-methoxy-3-methylphenyl)-3,7-dimethyl-2,4,6,8-nonatetraenoic acid, ethyl ester;
7,8-didehydroretinoic acid;
(E,E)-4-[2-methyl-4-(2,6,6-trimethyl-1-cyclohexen-1-yl)-1,3-butadienyl]benzoic acid;
(E)-4-[4-methyl-6-(2,6,6-trimethyl-1-cyclohexen-1-yl)-1,3,5-hexatrienyl]benzoic acid;
(all-E)-3,7-dimethyl-(3-thienyl)-2,4,6,8-nonatetraenoic acid;
(E,E,E)-3-methyl-7-(5,6,7,8-tetrahydro-5,5, 8,8-tetramethyl-2-naphthalenyl)-2,4,6-octatrienoic acid;
(E)-6-[2-(2,6,6-trimethyl-1-cyclohexen-1-yl)-ethenyl]-2-naphthalenecarboxylic acid;
(E,E,E)-7-(2,3-dihydro-1,1,3,3-tetramethyl-1H-inden-5-yl)-3-methyl-2,4,6-ocatrienoic acid;
(E)-4-[2-(2,3-dihydro-1,1,3,3-tetramethyl-1H-inden-5-yl)-1-propenyl]benzoic acid;
(E)-4-[2-(5,6,7,8-tetrahydro-5,5,8,8-tetramethyl-2-naphthalenyl-1-propenyl]benzoic acid;
(E)-4-[2-(5,6,7,8-tetrahydro-3-methyl-5,5,8,8-tetramethyl-2-naphthalenyl-1-propenyl]benzoic acid;
(E)-1,2,3,4-tetrahydro-1,1,4,4-tetramethyl-6-(1-methyl-2-phenylethenyl)naphthalene;
6-(1,2,3,4-tetrahydro-1,1,4,4-tetramethyl-6-naphthyl-2-naphthalenecarboxylic acid;
(E)-6-[2-(4-(ethylsulfonyl)phenyl]-1-methylethenyl]-1,2,3,4-tetrahydro-1,1,4,4-tetramethyl-naphthalene;
4-[(5,6,7,8-tetrahydro-5,5,8,8-tetramethyl-2-naphthalenyl)ethynyl]benzoic acid;
(E)-2-(1,1,4,4-tetramethyl-1,2,3,4-tetrahydronaphth-7-yl)-1-[4-tetrazol-5-yl)phenyl]-1-propene;
(E)-4-[2-(5,6,7,8-tetrahydro-7-hydroxy-5,5,8,8-tetramethyl-2-naphthalenyl)-1-propenyl]benzyl alcohol; etc.

The preferred retinoid is all-trans-retinoic acid.

The retinoid is applied topically in a composition such as a cream, an ointment, a lotion, a hydrogel, etc. or it can be incorporated into the adhesive layer of a bandage or dressing and/or located at the skin contacting adhesive surface of a bandage or dressing. The cream, lotion or ointment can be aqueous based, alcohol based, an oil in water emulsion, or a water in oil emulsion. Such composition will include additional pharmaceutically and cosmetically acceptable ingredients. For example, in addition to the particular vehicle, other ingredients such as solvents, surfactants, emulsifiers, penetration enhancing agents, stabilizers, preservatives, antioxidants, etc. can be included. The retinoid can also be coated onto or incorporated into a pressure sensitive adhesive composition such as the hydrocolloid containing adhesive described by Chen in U.S. Patent 3,339,546 or by Doyle et al. in U. S. Patent 4,551,490 or within the adhesive layer of the dressing described by Pawelchak et al. in U.S. Patent 4,538,603 by mixing with the other ingredients of the adhesive layer. The retinoid can also be coated onto the adhesive surface or incorporated into the adhesive layer of the compression bandage described by Frank in European Patent Application No. 370,789. The retinoid can also be located at the skin contacting adhesive surface and adjacent strata of the adhesive layer of a dressing or bandage according to the procedure described by Cilento et al. in European Patent Application No. 307,187. This procedure involves suspending the retinoid in mineral oil thickened with polyethylene and liquid petroleum base, i.e., Plastibase, casting the suspension onto release paper, and laminating the coated release paper to the adhesive layer of the bandage or dressing.

The retinoid can be present in the cream, ointment, lotion, solution or hydrogel composition at about 0.001% to about 0.1% by weight of the composition with amounts in the range of 0.01% to 0.05% being preferred. The retinoid can be present within the adhesive bandage or dressing at from about 0.2% to about 5%, preferably about 0.3% to about 1.2%, by weight of the adhesive layer. The retinoid when coated onto or present at the adhesive surface of the bandage or dressing can be at about 0.02 mg. to about 0.05 mg. preferably about 0.025 mg. of retinoid per sq. inch (6.452 cm²) of skin contacting adhesive surface. The composition can be applied on a daily basis, several times each day, or less frequently if formulated in a sustained release delivery system such as within the adhesive dressing where the retinoid is released over a longer period of time.

Patients who are comatose, diabetic, paraplegic, or otherwise suffering from serious impairment of the neural and vascular systems are at risk of developing pressure sores or ischemic ulcers. Such wounds tend to occur at parts of the body which are under compression such as the heels, elbows, hips, back, and buttock. According to this invention, by treating these areas of the patient's body with the retinoid containing composition prior to the development of such wounds, such as when redness is first noticed, the likelihood that the sore or ulcer will form is minimized.

In addition, there are a number of patients who suffer from skin stripping. Skin stripping is the removal of the outer epidermal layer caused by the removal of pressure sensitive adhesives.
For example, ostomates employ collection devices having a pressure sensitive adhesive coated faceplate for attachment to the body around their surgically created stoma. Such devices are removed daily and in some instances several times a day for disposal. This constant peeling of adhesive can result in removal or stripping of the epidermal layer. According to this invention, the effects of such skin stripping is eliminated or at least diminished by pretreating the skin with the retinoid containing composition or by including the retinoid within the adhesive composition.

The following examples are illustrative of this invention.

### EXAMPLE 1

The effect of pretreatment of skin with retinoids on the development of ischemic ulcers was determined by means of the following animal model.

### A. Compositions

Retinoid compositions were prepared containing 0.01%, 0.025%, and 0.05% by weight of all-trans retinoic acid in an aqueous cream formulation containing 31% water, 46% white petrolatum, about 11% isopropyl myristate, about 5% propylene glycol, surfactants, a preservative, and an antioxidant. A control cream formulation was also prepared of the same ingredients but without retinoic acid.

### B. Animal Model

Sixteen hairless guinea pigs (550 to 600 grams) were divided into 4 treatment groups: 0.05% retinoic acid, 0.025% retinoic acid, 0.01% retinoic acid, and control. Approximately one to two cc's of one of the cream formulations was applied to the dorsal area of each guinea pig daily for five days. Pretreatment was halted two days prior to surgery.

The animals were anesthetized and a 5 cm trans-scapular incision was made with a number 10 scapel. The skin was undermined using blunt dissection until a pocket was formed to accommodate the insert, a rubber tip of a plunger from a 10 ml. syringe. The insert was positioned over the mid-vertebral column distal to the scapula, at a site that was within the area pretreated with the retinoic acid or control formulations. A small rubber band tourniquet encircled the skin covering the syringe tip to prevent blood flow to the 5 cm² circle of skin over the insert. The incision was closed with wound staples.

Four hours after placement of the insert, the animals were reanesthetized. The incision was reopened, the rubber band was released, and the insert was removed by pulling an exposed suture attached to the rubber tip. All treatment and control sites were covered with a transparent polyurethane film/acrylic adhesive dressing (Bioclusive® from Johnson & Johnson) with the dressing extending beyond the 5 cm² circle but still adhering to portions of the animal's body that were pretreated with the retinoic acid or control formulations. The dressings were secured with an overwrap (Elasticon® from Johnson & Johnson).

### C. Microvascular Blood Cell Perfusion

A Laser Doppler Flowmeter (model Periflux PF2b from Perimed Inc., Stockholm, Sweden) was used to measure the microvascular blood cell perfusion through the site of ischemic injury which had been pretreated with the retinoic acid or control formulations both before and after surgery.

| Day | Control | 0.01% retinoic acid | 0.025% retinoic acid | 0.05% retinoic acid |
|---|---|---|---|---|
| 5 days prior to surgery | 69^{a}±9 | 66^{a}±8 | 65^{a}±5 | 64^{a}±2 |
| 2 days prior to surgery | 54^{a}±5 | 81^{ab}±8 | 72^{ab}±13 (3 animals) | 89^{b}±5 |
| just prior to surgery | 51^{a}±5 | 66^{a}±10 | 60^{a}±7 | 73^{a}±5 |
| 4 hours after surgery | 9^{a}±1 | 7^{a}±.6 | 13^{b}±1 | 10^{ab}±1 (3 animals) |
| 1 day after surgery | 51^{a}±5 | 66^{a}±10 | 60^{a}±7 | 73^{a}±5 |
| 5 days after surgery | 9^{a}±1 | 7^{a}±.6 | 13^{b}±1 | 10^{ab}±1 |
| 9 days after surgery | 43^{a}±8 | 89^{b}±4 | 75^{b}±9 | 90^{b}±3 |
| 14 days after surgery | 85^{b}±7 (3 animals) | 82^{b}±7 | 62^{a}±3 | 83^{b}±9 |
| Means in the same row sharing the same superscript (a or b) are not significantly different from each other. | | | | |

Pretreatment with the 0.05% retinoic acid formulation produced a significantly higher level of blood cell perfusion compared to the control.

The level of perfusion for all treatment groups is higher on days 5 and 9 post-surgery compared to the control, with an earlier return to normal perfusion for the group treated with 0.025% retinoic acid.

### D. Observations

Visual assessment of the ulcer site were made according to the grading system described by Shea, "Pressure Sores", Clinical Orthopaedics and Related Research, Number 12, October, 1975, pages 89 - 100:
Normal
Reepithelialization
Stage 1 - Erythema or edema
Stage 2 - Blistering or partial thickness broken skin
Stage 3 - White or black necrosis, full thickness
In addition on days 9 and 14 post-surgery the areas around the ulcer sites from which the adhesive dressings were being peeled were compared to determine whether the skin had been damaged by tape stripping.

| Day | Control | 0.01% retinoic acid | 0.025% retinoic acid | 0.05% retinoic acid |
|---|---|---|---|---|
| 1 day after surgery | Stage 2 | Stage 2 | Stage 2 | Stage 2 |
| 5 days after surgery | Stage 3 | Stage 1-2 | Stage 1-2 | Stage 1-2 |
| 9 days after surgery | Stage 3 stripping | Reepithelialization no stripping | Reepithelialization no stripping | Reepithelialization no stripping |
| 14 days after surgery | Stage 3 stripping | Normal no stripping | Normal no stripping | Normal no stripping |

### Example 2

The effect of pretreatment of skin with a retinoid containing adhesive dressing on the development of ischemic ulcers was determined by means of the following experiment.

### A. Dressing

The dressing employed consisted of a 1 mil (0.254 µm), backing film of polyurethane and a 10 mil. (2.54 µm) adhesive layer. The adhesive layer was a homogeneous blend containing on a weight percent basis about 16.3% butyl rubber, about 8% polyisobutylene, about 6% styrene-isoprene-styrene block copolymer, about 11.5% mineral oil, about 12.6% thickener (Pentalyn H), about 0.6% antioxidant, about 15% sodium carboxymethylcellulose, about 15% pectin, and about 15% gelatin.

A slurry was prepared containing mineral oil (45% by weight) thickened with Plastibase (55% by weight). All-trans retinoic acid was added at a desired concentration. The retinoic acid containing slurry was coated using a Werner Mathis Coating machine onto silicone coated release paper at a thickness of about 0.5 mils (0.127 µm).

The coated release paper was then laminated to the surface of the adhesive dressing described above and allowed to set for several hours. The thickened mineral oil retinoic acid slurry was gradually absorbed into the adhesive layer of the dressing. The concentration of all-trans retinoic acid was about 0.025 mg. per square inch (6.542 cm²) of adhesive surface.

A control dressing was prepared in which the thickened mineral slurry without added retinoic acid was coated onto release paper at a thickness of about 0.5 mils. The release paper was then laminated to the adhesive layer of the dressing described above.

### B. Animal Model

As described in Example 1. The dressings were applied to the dorsal area of each guinea pig and left in place for five days.

### C. Microvascular Blood Cell Perfusion

The technique employed was that described in Example 1.

| Day | Control | Dressing Without Retinoic Acid | Dressing Having Retinoic Acid At The Adhesive Surface At a Concentration Of 0.025 mg per Square Inch |
|---|---|---|---|
| 5 days prior to surgery | 54^{a}±3 | 49^{a}±3 | 56^{a}±3 |
| day of surgery | 50^{a}±6 | 60^{a}±5 | 59^{a}±7 |
| 5 days after surgery | 0^{a}±0 | 8^{a}±8 | 12^{a}±6 |
| 10 days after surgery | 0^{a}±0 | 19^{a}±12 | 67^{b}±11 |
| 15 days after surgery | 35^{a}±13 | 78^{a}±17 | 69^{a}±11 |
| 20 days after surgery | 36^{a}±18 | 74^{a}±11 | 46^{a}±3 |
| Means in the same row sharing the same superscript (a or b) are not significantly different from each other. | | | |

### D. Observations

Pretreatment with the dressing having a retinoic acid concentration of 0.025 mg. per square inch of adhesive surface produced a significantly higher level of blood cell perfusion compared to the control and the dressing without retinoic acid.

Pretreatment with the dressing containing all-trans retinoic acid at the concentration of 0.025 mg. per square inch of adhesive surface prior to ischemic insult minimized skin damage from a tourniquet induced ischemia. Pretreatment with the control dressing (no retinoic acid) or no pretreatment produced substantial amounts of necrosis resembling stage III pressure sores.

## Claims

1. Use of a retinoid for the manufacture of a medicament for preventing or lessening the skin damaging effects of pressure sores and ischemic ulcers by pretreatment of areas of the body susceptible to the formation of pressure sores and ischemic ulcers.

2. The use as defined in claim 1 wherein said medicament is a cream, ointment, lotion, solution, or hydrogel composition.

3. The use as defined in claim 2 wherein said retinoid is present in the composition at 0.001% to 0.1% by weight of said composition.

4. The use as defined in claim 3 wherein said retinoid is present in the composition at from 0.01% to 0.05% by weight of said composition.

5. The use as defined in claim 1 wherein said retinoid is incorporated within the adhesive layer of a bandage or dressing or is coated onto the skin contacting adhesive surface of a bandage or dressing or is located at the skin contacting adhesive surface and adjacent strata of a bandage or dressing.

6. The use as defined in claim 5 wherein said retinoid is present at 0.02 mg. to 0.05 mg. per square inch (6.452 cm²) of the skin contacting adhesive surface of said bandage or dressing.

7. The use as defined in claim 6 wherein said retinoid is present at about 0.025 mg. per square inch (6.452 cm²) of the skin contacting adhesive surface of said bandage or dressing.

8. The use as defined in claim 5 wherein said retinoid is incorporated within the adhesive layer of said bandage or dressing at from 0.2% to 5% by weight of said adhesive layer.

9. The use as defined in claim 8 wherein retinoid is at from 0.3% to 1.2% by weight of said adhesive layer.

10. The use as defined in any preceding claim wherein said retinoid is all-trans retinoic acid.

11. Use of a retinoid for the manufacture of a medicament for preventing or lessening skin damage due to the stripping of the epidermal layer caused by the removal of adhesives from the body by pretreatment of the areas of the body from which an adhesive will be removed.

12. The use as defined in claim 11 wherein said medicament is a cream, ointment, lotion, solution, or hydrogel composition.

13. The use as defined in claim 12 wherein said retinoid is present in the composition at 0.001% to 0.1% by weight of said composition.

14. The use as defined in claim 13 wherein said retinoid is present in the composition at from 0.01% to 0.05% by weight of said composition.

15. The use as defined in claim 11 wherein said retinoid is incorporated within the adhesive layer of a dressing or bandage or is coated onto the skin contacting adhesive surface of a bandage or dressing or is located at the skin contacting adhesive surface and adjacent strata of the bandage or dressing.

16. The use as defined in claim 15 wherein said retinoid is present at 0.02 mg. to 0.05 mg. per square inch (6.452 cm²) of the skin contacting adhesive surface of said bandage or dressing.

17. The use as defined in claim 16 wherein said retinoid is present at about 0.025 mg. per square inch (6.452 cm²) of the skin contacting adhesive surface of said bandage or dressing.

18. The use as defined in claim 15 wherein said retinoid is incorporated within the adhesive layer of said bandage or dressing at from 0.2% to 5% by weight of said adhesive layer.

19. The use as defined in claim 18 wherein said retinoid is at from 0.3% to 1.2% by weight of said adhesive layer.

20. The use as defined in any one of claims 11-19 wherein said retinoid is all-trans retinoic acid.

## Patentansprüche

1. Verwendung von Retinoid zur Herstellung eines Medikaments zur Verhinderung oder Minderung hautschädigender Wirkungen von Druckverletzungen und ischämischen Geschwüren durch Vorbehandlung der Körperstellen, die für die Entstehung von Druckverletzungen und ischämischen Geschwüren anfällig sind.

2. Verwendung nach Anspruch 1, wobei das Medikament eine Creme, Salbe, Lotion, Lösung oder eine Hydrogel-Zusammensetzung ist.

3. Verwendung nach Anspruch 2, wobei das Retinoid in der Zusammensetzung mit 0,001 bis 0,1 Gew.-% der Zusammensetzung vorhanden ist.

4. Verwendung nach Anspruch 3, wobei das Retinoid in der Zusammensetzung mit 0,01 bis 0,05 Gew.-% der Zusammensetzung vorhanden ist.

5. Verwendung nach Anspruch 1, wobei das Retinoid in der Klebstoffschicht einer Bandage oder eines Verbands enthalten ist oder auf die die Haut berührende Klebstoffoberfläche der Bandage oder des Verbands aufgebracht wird oder sich an der die Haut berührende Klebstoffoberfläche und angrenzenden Schichten der Bandage oder des Verbands befindet.

6. Verwendung nach Anspruch 5, wobei 0,02 bis 0,05 mg/inch² (6,452 cm²) des Retinoids in der die Haut berührenden Klebstoffoberfläche der Bandage oder des Verbands vorhanden sind.

7. Verwendung nach Anspruch 6, wobei etwa 0,025 mg/inch² (6,452 cm²) des Retinoids in der die Haut berührenden Klebstoffoberfläche der Bandage oder des Verbands vorgesehen sind.

8. Verwendung nach Anspruch 5, wobei 0,2 bis 5 Gew.-% bezogen auf die Klebstoffschicht Retinoid in der Klebstoffschicht der Bandage oder des Verbands enthalten sind..

9. Verwendung nach Anspruch 8, wobei das Retinoid 0,3 bis 1,2 Gew.-% der Klebstoffschicht ausmacht.

10. Verwendung nach einem der vorstehenden Ansprüche, wobei das Retinoid all-transRetinoesäure ist.

11. Verwendung eines Retinoids zur Herstellung eines Medikaments zur Verhinderung oder Minderung der Hautschädigung durch Abziehen der Epidermisschicht durch das Entfernen von Heftpflaster vom Körper durch Vorbehandlung der Körperstellen, von denen das Heftpflaster entfernt wird.

12. Verwendung nach Anspruch 11, wobei das Medikament eine Creme, Salbe, Lotion, Lösung oder eine Hydrogel-Zusammensetzung ist.

13. Verwendung nach Anspruch 12, wobei 0,001 bis 0,1 Gew.-% bezogen auf die Zusammensetzung Retinoid in der Zusammensetzung vorhanden sind.

14. Verwendung nach Anspruch 13, wobei 0,01 bis 0,05 Gew.-% bezogen auf die Zusammensetzung Retinoid in der Zusammensetzung mit vorhanden sind.

15. Verwendung nach Anspruch 11, wobei das Retinoid in der Klebstoffschicht eines Verbands oder einer Bandage enthalten ist oder auf die die Haut berührende Klebstoffoberfläche der Bandage oder des Verbands aufgebracht wird oder sich auf der die Haut berührende Klebstoffoberfläche und angrenzenden Schichten der Bandage oder des Verbands befindet.

16. Verwendung nach Anspruch 15, wobei 0,02 bis 0,05 mg/inch² (6,452 cm²) des Retinoids in der die Haut berührenden Klebstoffoberfläche der Bandage oder des Verbands vorhanden sind.

17. Verwendung nach Anspruch 16, wobei etwa 0,025 mg/inch² (6,452 cm²) des Retinoids in der die Haut berührenden Klebstoffoberfläche der Bandage oder des Verbands vorhanden sind.

18. Verwendung nach Anspruch 15, wobei 0,2 bis 5 Gew.-% bezogen auf die Klebstoffschicht Retinoid in der Klebstoffschicht der Bandage oder des Verbands enthalten sind.

19. Verwendung nach Anspruch 18, wobei das Retinoid 0,3 bis 1,2 Gew.-% der Klebstoffschicht ausmacht.

20. Verwendung nach einem der Ansprüche 11 bis 19, wobei das Retinoid all-trans-Retinoesäure ist.

## Revendications

1. Utilisation d'un rétinoïde pour la fabrication d'un médicament permettant de prévenir ou d'amoindrir les effets dommageables pour la peau des escarres et des ulcères ischémiques par prétraitement de surfaces du corps susceptibles à la formation d'escarres et d'ulcères ischémiques.

2. Utilisation selon la revendication 1, dans laquelle ledit médicament est une composition de crème, de pommade, de lotion, de solution ou d'hydrogel.

3. Utilisation selon la revendication 2, dans laquelle ledit rétinoïde représente de 0,001% à 0,1% du poids de ladite composition.

4. Utilisation selon la revendication 3, dans laquelle ledit rétinoïde représente de 0,01% à 0,05% du poids de ladite composition.

5. Utilisation selon la revendication 1, dans laquelle ledit rétinoïde est incorporé à la couche adhésive d'un bandage ou d'un pansement, ou est étalé sur la surface adhésive de contact avec la peau d'un bandage ou d'un pansement, ou est placé à la surface adhésive de contact avec la peau et dans les couches adjacentes d'un bandage ou d'un pansement.

6. Utilisation selon la revendication 5, dans laquelle ledit rétinoïde est présent à raison de 0,02 mg à 0,05 mg par pouce carré (6,452 cm²) de la surface adhésive de contact avec la peau dudit bandage ou dudit pansement.

7. Utilisation selon la revendication 6, dans laquelle ledit rétinoïde est présent à raison d'environ 0,025 mg par pouce carré (6,452 cm²) de la surface adhésive de contact avec la peau dudit bandage ou dudit pansement.

8. Utilisation selon la revendication 5, dans laquelle ledit rétinoïde est incorporé au sein de la couche adhésive dudit bandage ou dudit pansement, à raison de 0,2% à 5% du poids de ladite couche adhésive.

9. Utilisation selon la revendication 8, dans laquelle ledit rétinoïde représente de 0,3% à 1,2% du poids de ladite couche adhésive.

10. Utilisation selon l'une quelconque des revendications précédentes, dans laquelle ledit rétinoïde est l'acide rétinoïque entièrement sous forme trans.

11. Utilisation d'un rétinoïde pour la fabrication d'un médicament permettant de prévenir ou d'amoindrir les dommages cutanés dûs à l'arrachement de la couche épidermique causé par le décollement d'adhésifs du corps, par prétraitement des surfaces du corps desquelles un adhésif va être décollé.

12. Utilisation selon la revendication 11, dans laquelle ledit médicament est une composition de crème, de pommade, de lotion, de solution ou d'hydrogel.

13. Utilisation selon la revendication 12, dans laquelle ledit rétinoïde représente de 0,001% à 0,1% du poids de ladite composition.

14. Utilisation selon la revendication 13, dans laquelle ledit rétinoïde représente de 0,01% à 0,05% du poids de ladite composition.

15. Utilisation selon la revendication 11, dans laquelle ledit rétinoïde est incorporé à la couche adhésive d'un bandage ou d'un pansement, ou est étalé sur la surface adhésive de contact avec la peau d'un bandage ou d'un pansement, ou est placé à la surface adhésive de contact avec la peau et dans les couches adjacentes d'un bandage ou d'un pansement.

16. Utilisation selon la revendication 15, dans laquelle ledit rétinoïde est présent à raison de 0,02 mg à 0,05 mg par pouce carré (6,452 cm²) de la surface adhésive de contact avec la peau dudit bandage ou dudit pansement.

17. Utilisation selon la revendication 16, dans laquelle ledit rétinoïde est présent à raison d'environ 0,025 mg par pouce carré (6,452 cm²) de la surface adhésive de contact avec la peau dudit bandage ou dudit pansement.

18. Utilisation selon la revendication 15, dans laquelle ledit rétinoïde est incorporé au sein de la couche adhésive dudit bandage ou dudit pansement, à raison de 0,2% à 5% du poids de ladite couche adhésive.

19. Utilisation selon la revendication 18, dans laquelle ledit rétinoïde représente de 0,3% à 1,2% du poids de ladite couche adhésive.

20. Utilisation selon l'une quelconque des revendications 11 - 19, dans laquelle ledit rétinoïde est l'acide rétinoïque entièrement sous forme trans.
